# EUROPEAN PATENT SPECIFICATION

(11) **EP 3 355 794 B1**
(45) Date of publication and mention of the grant of the patent: **02.12.2020**
(21) Application number: 16774488.7
(22) Date of filing: 29.09.2016
(51) Int. Cl.: A61B 6/12, A61B 6/00

(54) **X-RAY IMAGING OF AN OBJECT WITH THREE-DIMENSIONAL LOCALIZATION OF AN INTERVENTIONAL DEVICE**
RÖNTGENBILDGEBUNG EINES OBJEKTS MIT DREIDIMENSIONALER LOKALISIERUNG EINER INTERVENTIONELLEN VORRICHTUNG
IMAGERIE PAR RAYONS X D'UN OBJET AVEC UNE LOCALISATION TRIDIMENSIONNELLE D'UN DISPOSITIF D'INTERVENTION

(30) Priority: 30.09.2015 EP 15187548
(43) Date of publication of application: 08.08.2018
(73) Proprietor: Koninklijke Philips N.V., 5656 AG Eindhoven (NL)
(72) Inventor: SCHAEFER, Dirk, 5656 AE Eindhoven (NL); GRASS, Michael, 5656 AE Eindhoven (NL); KOEHLER, Thomas, 5656 AE Eindhoven (NL)
(74) Representative: Versteeg, Dennis John
(86) International application number: PCT/EP2016/073164
(87) International publication number: WO 2017/055383

(56) References cited:
- US-A1- 2001 036 245
- US-A1- 2006 115 054
- US-A1- 2009 052 614
- US-A1- 2013 272 572
- US-A1- 2015 031 986

## Description

### FIELD OF THE INVENTION

The invention relates to an X-ray imaging apparatus for determining a position of an interventional device within an object receiving space. The present invention further relates to a corresponding method, a corresponding system, a corresponding computer program element and a corresponding computer-readable medium.

### BACKGROUND OF THE INVENTION

X-ray imaging apparatuses and systems are used in operating rooms environments and/or for other purposes. Usually, an X-ray source is provided, which emits X-ray radiation. At least a part of the X-ray radiation provided by the X-ray source penetrates an object of interest, which may be arranged between the X-ray source and an X-ray detector. X-ray radiation influenced by the object of interest may be detected by the X-ray detector. The X-ray detector may provide a detection signal indicating an image of the object of interest. The image may represent a two-dimensional image of the object of interest. Depth information along a viewing direction of the X-ray radiation from the X-ray source to the X-ray detector may be lost due to its projective nature. Nevertheless, this information may be valuable at least for some applications, in particular to navigate an interventional device within the object of interest, for instance following a three-dimensional roadmap during an interventional procedure. To tackle this problem, document C. Haase et al., "3D ablation catheter localization using individual C-arm X-ray projections", Phys. Med. Biol. 59, 6959-6977, 2014, suggest a tracking method and a registration method of a single X-ray radiation projection, where the perspective magnification is exploited.

US2015/031986A1 discloses a phase contrast x-ray imaging system with a grating-based interferometer for recording an interventional medical instrument in a subject, wherein the medical instrument includes at least one component which exhibits a strong small angle scattering of x-rays.

### SUMMARY OF THE INVENTION

There may be a need for an enhanced determination of a position of an interventional device within an object receiving space of an X-ray imaging apparatus.

The object of the present invention is solved by the subject-matter of the independent claims, wherein further embodiments are incorporated in the dependent claims. It should be noted that the following described aspects of the invention apply also to the apparatus, the system, the computer program element and the computer-readable medium.

According to a first aspect of the invention, an X-ray imaging apparatus for determining a position of an interventional device within an object receiving space is provided. The X-ray imaging apparatus comprises an X-ray source for generating an X-ray radiation, an X-ray detector, an object receiving space for arranging an object of interest for X-ray imaging, an interferometer for creating interference pattern, and a processing unit. The object receiving space is arranged between the X-ray source and the X-ray detector. The interferometer is arranged between the object receiving space and the X-ray source and/or the object receiving space and the X-ray detector. The X-ray detector is configured to detect X-ray radiation influenced by the interferometer and in response thereto provide a detector signal indicating an image. The processing unit is configured to identify, based on the detector signal, the interventional device within the object receiving space. The processing unit is further configured to calculate, based on the detector signal, a visibility loss signal for the identified interventional device. The processing unit is further configured to calculate, based on the visibility loss signal, a position signal representing the position of the interventional device within the object receiving space.

It is to be noted, that the process unit is configured to calculate, based on the detector signal, a visibility loss signal for the identified interventional device. Accordingly, the visibility loss signal may indicate a visibility loss caused by the identified interventional device.

It is further noted, that the processing unit is configured to calculate, based on the visibility loss signal, a position signal representing the position of the interventional device within the object receiving space.

It has been found, that a distance between the interferometer and the interventional device, arranged at the object receiving space, depends on the visibility loss caused by the interventional device and/or the visibility loss signal.

As a result, the position signal provided by the processing unit indicates also the information about the depth of the interventional device within the object receiving space. Consequently, the position signal may be used to identify the depth of the interventional device along a viewing direction of an X-ray radiation from the X-ray source to the detector, or vice versa. This information about the depth of the interventional device allows determining a three-dimensional position of the interventional device within the object receiving space.

Thus, an in particular as a result, the visibility loss signal may serve as the basis for calculating a "depth-position" of the interventional device within the object receiving space.

In an example according to the present invention, the interferometer may comprise a grating. The grating may be an absorption grating, a phase grating or a combination thereof.

In another example according to the present invention, seen from the X-ray source to the X-ray detector, the X-ray radiation provided by the X-ray source is influenced by the interferometer. The X-ray radiation may be further influenced by an object of interest, which may be arranged at the object receiving space.

In an example of the present invention, the processing unit is configured to identify an interventional device, in particular a catheter, within an object receiving space arranged between an X-ray source and an X-ray detector. In particular, the processing unit may be configured to use the detector signal to provide a two-dimensional position within an image, which may be provided based on the detector signal. Accordingly, an identification of an interventional device a the object receiving space may relate to a two-dimensional position identification of the interventional device within an image plane of the image relating to the object receiving space or an object of interest arranged at the object receiving space.

In an example of the present invention, the processing unit is configured to calculate, based on the detector signal, an absorption signal indicating an absorption of the X-ray radiation provided from the X-ray source in the direction to the detector.

In another example according to the present invention, the position of the interventional device within the object receiving space may relate to a depth of the interventional device within the object receiving space and/or an object of interest, in case the object of interest is arranged in the object receiving space.

As a result, the processing unit may be configured to calculate a three-dimensional position of the interventional device within the object receiving space based on the detector signal and/or the visibility loss signal.

As a further result, depth information along the viewing direction of the X-ray projection may be provided and thus may be used for following a roadmap with an interventional device through an object of interest.

According to an exemplary embodiment of the present invention, the apparatus further comprises a display unit. Further, the processing unit may be configured to calculate, based on the detector signal, an image signal representing at least one part of the object of interest. Furthermore, the processing unit may be configured to calculate, based on the image signal and the position signal a display signal representing the image of the object of interest and a notation of the position of the interventional device. Further, the display unit may be configured to display a display image based on the display signal.

As a result, depth information of the interventional device may be recognizable by a corresponding notation at the display image.

In another example according to the present invention, the notation of the position of the interventional device may represent a relative position with respect to the object of interest or the object receiving space.

As a result, the display image may show the image of at least a part of the object of interest and may further show a notation of the position of the interventional device, in particular at the object of interest.

In another example according to the present invention, the position of the interventional device relates to a depth of the interventional device at the object of interest.

In a further example according to the present invention, the position of the interventional device relates to a three-dimensional position of the interventional device, in particular within the object receiving space or at the object of interest.

As a further result, the display image may show a two-dimensional /or image of the interventional device at a two-dimensional image of at least part of the object of interest. Furthermore, the information about the third dimension of the interventional device, in particular with respect to the object of interest, may be shown and therefore may be provided at the display image. As a result, the display image may provide three-dimensional information about an interventional device in conjunction with an image of at least a part of the object of interest.

According to a further exemplary embodiment according to the present invention, the processing unit may be configured to calculate, based on the visibility loss signal, a distance signal representing a distance between a section of the interventional device and the interferometer or an element fixed thereto. The processing unit may be further configured to calculate, based on the distance signal, the position signal representing the position of the interventional device within the object receiving space.

As a result, a physician may receive further depth information about a particular section of the interventional device during an interventional procedure.

In another example according to the present invention, calculating the distance signal may be interpreted as a sub-feature of calculating the position signal. Accordingly, the respective configuration of the processing unit may be interpreted as a sub-configuration of the calculation-configuration of the processing unit.

As a result, the section of the interventional device, thus in particular a preferred section of higher interest, may provide the basis for identifying a depth or a three-dimensional position of the interventional device within the object receiving space and/or an object arranged at the object receiving space.

According to a further exemplary embodiment according to the present invention, the notation of the position comprises a highlight-notation. Further the processing unit may be configured to determine a colour of the highlight-notation based on the position signal. Furthermore, the processing unit may be configured to calculate the display signal, such that the highlight-notation highlights at least a part of the imaged interventional device at the display image with the colour determined for the highlight-notation.

As a result, the interventional device imaged at the display image may be highlighted, such that a physician can immediately identify the position of the interventional device.

According to a further exemplary embodiment according to the present invention, the notation of the position comprises a number-notation representing a value of the position of the interventional device. Further, the processing unit may be configured to calculate the display signal, such that the display image comprises the number-notation.

As a result, the value of the position of the interventional device, in particular relating to its depth, may be directly shown at the display image and thus being immediately recognized by a physician using the apparatus.

According to a further exemplary embodiment according to the present invention, the notation of the position comprises a slider and a slider bar. Further, the slider bar may represent a scale between a predefined minimum value of the position signal and a predefined maximum value of the position signal. Furthermore, the processing unit may be configured to determine, on the basis of the position signal, a slider position of the slider at the slider bar. Further, the processing unit may be configured to calculate the display signal, such that the display image also shows the slider bar and the slider at the slider position.

As a result, a physician may receive with a look at the display image quantitative information about the depth of the interventional device.

According to a further exemplary embodiment according to the present invention, the processing unit is configured to receive a spot signal. Further, the processing unit may be configured to save, for each time of a reception of the spot signal, a position of the section of the interventional device and the colour of the highlight-notation for the section as an interventional device spot. Furthermore, the processing unit may be configured to calculate the display image, such that the display image comprises each interventional device spot.

As a result, the display image may comprise several highlight-notations. Each highlight-notation may be arranged at the position of the interventional device for the time, when the spot signal was received. Thus, a physician may mark relevant positions of the highlight-notations to keep track of passed interventional device positions.

According to a second aspect of the present invention, an X-ray system for determining a position of an interventional device within an object receiving space is provided. The X-ray system comprises an apparatus according to one of the examples as explained above, and an interventional device. The interventional device comprises a structure comprising a structure size of less than twice of a grating pitch of a grating of the interferometer.

As a result, the interventional device may cause a higher contrast at the dark-field image signal component of the detector signal.

As a further result, the interventional device may be well identified on the basis of the visibility loss signal for the interventional device.

According to an exemplary embodiment of the X-ray system according to the present invention, the interventional device comprises the structure having the structure size at a section of the interventional device.

As a result, the section having the structure size may cause a higher contrast at the dark-field image signal component of the detector signal than the remaining interventional device. Thus, the section having the structure size may be well recognized.

In another example of the X-ray system according to the present invention, the interventional device comprises the structure having the structure size just at the section of the interventional device.

As a result, the section of the interventional device may be well identified, in particular on the basis of the visibility loss signal.

In another example according to the present invention, the section of the interventional device may be arranged at an end section of the interventional device.

In a further example according to the present invention, the section of the interventional device may relate to a tip-section of the interventional device.

According to a further exemplary embodiment of the X-ray system according to the present invention, the interventional device comprises a sheath for forming the structure having the structure size. The sheath may comprise an X-ray absorption characteristic that has a maximum variance of 20% to an X-ray absorption characteristic of water.

As a result, the interventional device, in particular its sheath, may provide a very low influence on the absorption image signal component of the detector signal. Thus, the absorption image signal component may be used to map the object of interest with very low irritation of the interventional device.

As a result, the section of the interventional device has a low influence on an absorption image signal component of the detector signal.

As a further result, the absorption image signal component may be used to calculate an image of the object of interest, preferably with low irritation of the interventional device.

As a further result, the section having the structure size may still be very well identified on the basis of the visibility loss signal for the interventional device.

According to a further exemplary embodiment of the X-ray system according to the present invention, the processing unit is configured to calculate, based on an absorption-component of the detector signal and/or a phase-contrast-component of the detector signal, an image signal representing at least one part of the object of interest at the object receiving space. Further, the processing unit may be configured to calculate, based on identified interventional device, a device signal representing at least one part of the interventional device. Furthermore, the processing unit may be configured to calculate the display signal on the base of the image signal and the device signal, such that the interventional device is represented as an overlay to the representation of the object of interest at the display image.

As a result, the absorption characteristic of the interventional device may have a low effect at the visibility loss signal. Thus, the interventional device may still be very well identified based on the visibility loss signal.

According to a further exemplary embodiment of the X-ray system according to the present invention, the system comprises a control unit for controlling the interventional device. Further, the interventional device may comprise an actuator at a section of the interventional device. Furthermore, the control unit may be configured to activate the actuator. Further, the control unit may be configured to send a spot signal to the processing unit upon the activation of the actuator.

As a result, a spot signal received by the processing unit may create an interventional device spot, in particular as a highlight-notation, at the current position of the interventional device. The interventional device spots may remain at the display image allowing a physician to keep track of the past three-dimensional positions of the interventional device.

According to a third aspect of the present invention, a method for determining a position of an interventional device within an object receiving space is provided. The method comprises the following steps:
a) emitting an X-ray radiation from an X-ray source in direction to an X-ray detector, wherein an object receiving space is arranged between the X-ray source and the X-ray detector, and wherein an interferometer is arranged between the object receiving space and the X-ray detector or the object receiving space and the X-ray source;
b) providing a detector signal based on X-ray radiation detected by the detector, wherein the detected X-ray radiation is influenced by the interferometer and/or an object of interest arrangeable at the object receiving;
c) identifying, based on the detector signal, an interventional device within the object receiving space;
d) calculating, based on the detector signal, a visibility loss signal for the identified interventional device;
e) calculating, based on the visibility loss signal, a position signal representing a position of the interventional device within the object receiving space.

With respect to the order of the steps, it is noted that step a) may be performed in parallel to step b). Steps a) and b) may be performed before or in parallel to step c). Step c) may be performed before step d), in parallel, or vice versa. Step e) may be performed after step d).

According to a fourth aspect of the invention, a computer program element is provided, which, when being executed by the processing unit, is adapted to perform the method steps described above.

According to a fifth aspect of the present invention, a computer-readable medium having stored thereon a program element is provided, which, when being executed by the processing unit, is adapted to carry out the method described above.

According to an aspect of the present invention, an apparatus and a method for a depth localization of a catheter within an object of interest, arranged at an object receiving space of an X-ray imaging apparatus, is provided. An X-ray imaging apparatus comprises an X-ray source and an X-ray detector. Between the X-ray source and the X-ray detector, at least one interferometer is arranged. Preferably, the X-ray system comprises at least three gratings. A grating may also be referred to as an X-ray grating. Further, an object receiving space is arranged between the X-ray source and the X-ray detector. Between the X-ray source and the object receiving space, a first absorption grating may be provided, in order to provide coherent X-ray radiation to the object receiving space. Between the object receiving space and the detector, a series of the remaining two gratings is preferably provided. In particular, a first grating of the series of gratings is a phase grating. The phase grating is provided between the object receiving space and the detector, wherein between the phase grating and the detector, the second grating of the series of gratings, namely a further absorption grating configured as an analyzer grating, is provided.

The X-ray detector is configured to detect X-ray radiation, which has been influenced by the gratings of the X-ray system and in particular by an object of interest, which may be arranged at the object receiving space. The X-ray detector is configured to provide a detector signal. The detector signal indicates an image of the object of interest and/or any other object arranged at the object receiving space, for instance an interventional device. On the basis of the detector signal, three detector signal components may be derived. First, an absorption signal component may be calculated, wherein the absorption signal component represents an absorption image of at least one object arranged at the object receiving space. The second signal component relates to a phase-contrast detector signal component, which may be calculated on the basis of the detector signal and represents a differential phase-contrast image of the at least one object. The third component is the dark-field signal component of the detector signal, which may be calculated on the basis of the detector signal and which represents a dark-field image of the at least one object.

The aforementioned images represent two-dimensional images. A depth information along the viewing direction of the X-ray projection image is lost due to its protective nature. Nevertheless, this information is valuable for some applications, in particular to navigate a catheter along a three-dimensional roadmap of an object of interest during an interventional procedure through the object of interest.

It has been found, that the visibility loss of an object depends on a distance between the object, in particular the interventional device, and one of the gratings, in particular one of the phase gratings, in particular the second phase grating, which is next to the object receiving space and arranged between the detector and the object receiving space. Accordingly, a distance may be calculated on the basis of the dark-field image signal component of the detector signal, which may be also called as a visibility loss signal. Having knowledge about the distance between the object, in particular the interventional device, and the grating, in particular the second phase grating, any other depth information may be calculated on the this basis, since the grating, in particular the second phase grating, may have a fixed or known distance to the X-ray detector, the X-ray source and/or the object receiving space. Correspondingly, a three-dimensional position of the object, in particular an interventional device, preferably formed by a catheter, within the object receiving space may be calculated. It is to be noted, that the catheter may just be one example of an interventional device, which may be arranged at the object receiving space.

As an effect, a three-dimensional tracking of the interventional device, in particular of a catheter, may be enabled from the detector signal.

The depth information of the interventional device, in particular based on the distance between the interventional device and one of the gratings, in particular one of the phase gratings, may also be used in a colour overlay notation, in particular by detected ablation points by memorizing the depth information of the interventional device, in particular its tip, at the respective moment. This information may be represented as an overlay image to the absorption image of the object of interest, which may be displayed with a monitor of the X-ray system.

Further, the interventional device, preferably the catheter, may comprise a sheath, in particular at its tip section, which comprises a microstructure with a structure distance comparable to the grating distance of the interferometer and/or an attenuation coefficient comparable to water or biological tissue. When a phase-contrast X-ray imaging system is used for interventional guidance, the catheter may not be shown up in the absorption image. Instead, the catheter, in particular its sheath, will deliver a strong signal at the dark-field image or the visibility loss image. This is basically due to small angle scattering. It can be detected, tackled and/or segmented from the dark-field image or the visibility loss signal, respectively. In an example, the sheath of the catheter, and/or a sample image representing the respective section of the catheter, may be overlaid to an absorption image of the object of interest.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically illustrates an X-ray imaging apparatus according to an exemplary embodiment of the invention.
Fig. 2 shows a diagram illustrating the dependence of the visibility loss from the distance between an interventional device and a phase grating of the X-ray imaging apparatus.
Fig. 3 schematically illustrates an X-ray imaging apparatus according to a further exemplary embodiment of the invention.
Fig. 4 schematically illustrates an X-ray imaging apparatus according to a further exemplary embodiment of the invention.
Fig. 5 schematically illustrates an X-ray imaging apparatus according to a further exemplary embodiment of the invention.
Fig. 6 schematically illustrates an X-ray imaging apparatus according to a further exemplary embodiment of the invention.
Fig. 7 schematically illustrates an example of a display image showing a part of an object of interest and an interventional device.
Fig. 8 schematically illustrates a further example of a display image showing a part of an object of interest and an interventional device.
Fig. 9 schematically illustrates a further example of a display image showing a part of an object of interest and an interventional device.
Fig. 10 schematically illustrates an example of the interventional device in a cross sectional view.
Fig. 11 schematically illustrates an example of the interventional device in a different cross sectional view.
Fig. 12 schematically illustrates a further example of a display image showing a part of an object of interest and an interventional device.
Fig. 13 schematically illustrates an X-ray imaging system according to an exemplary embodiment of the invention.
Fig. 14 schematically illustrates a method according to an exemplary embodiment of the present invention.

### DETAILED DESCRIPTION OF THE EMBODIMENTS

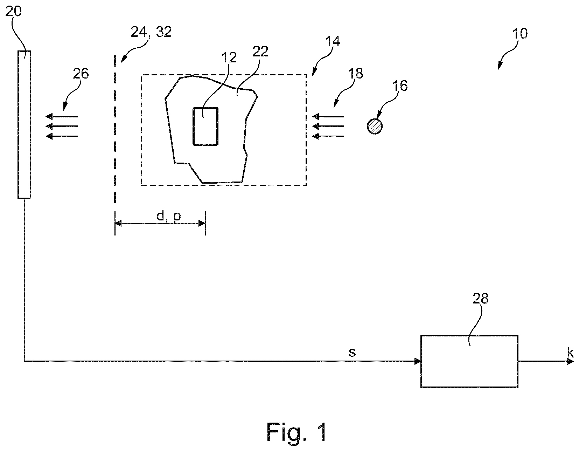

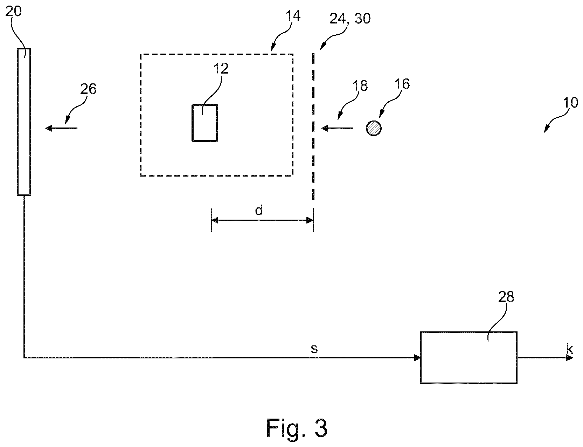

Fig. 1 schematically illustrates an X-ray imaging apparatus 10 for determining a position p of an interventional device 12 within an object receiving space 14 according to an exemplary embodiment of the present invention. The X-ray imaging apparatus 10 comprises an X-ray source 16 for generating an X-ray radiation 18, an X-ray detector 20, an object receiving space 14 for arranging an object of interest 22 for X-ray imaging, an interferometer 24 for creating interference pattern, and a processing unit 28. The object receiving space 14 is arranged between the X-ray source 16 and the X-ray detector 20. The interferometer 24 is arranged between the object receiving space 14 and the X-ray detector 20. The interferometer may alternatively be arranged, as exemplarily shown in Fig. 3, between the object receiving space 14 and the X-ray source 16. The X-ray detector 20 is configured to detect X-ray radiation 26 influenced by the interferometer and in response thereto provide a detector signal s indicating an image. The processing unit 28 is configured to identify, based on the detector signal s, the interventional device 12 within the object receiving space 14. The processing unit 28 is further configured to calculate, based on the detector signal s, a visibility loss signal for the interventional device 12. The processing unit 28 is further configured to calculate, based on the visibility loss signal, a position signal k representing a position p of the interventional device 12 within the object receiving space 14.

In an example, the object receiving space is configured for arranging the interventional device 12, in particular within an object of interest 22 at the object receiving space 14.

In an example, the position p of the interventional device 12 may relate to a depth d of the interventional device 12 within the object receiving space 14. The depth d may be a distance between the interventional device 12 and the interferometer 24, or an element of the apparatus 10 fixed thereto.

In an example, the position p of the interventional device 12 may relate to a depth of the interventional device 12 within an object of interest 22, which may be arranged at the object receiving space 14.

In an example, the position p may comprise a depth-component representing the depth d of the interventional device 12 within the object receiving space 14.

As a result, the position signal k provided by the processing unit 28 indicates at least also information about the depth d of the interventional device 12 within the object receiving space 14. This depth information may relate to a fixed element of the apparatus 10. Consequently, the position signal k may be used to identify the depth of the interventional device 12 along a viewing direction of an X-ray radiation from the X-ray source 16 to the detector 20, or vice versa. This information about the depth of the interventional device 12 allows to more accurately navigate the interventional device 12 within the object receiving space 14. In particular, the interventional device 12 may be more accurately navigated along a three-dimensional roadmap of the object of interest 22 during an interventional procedure, which is carried out at the object of interest 22 at the object receiving space 14.

In an example, the interventional device 12 may relate to a catheter, a medical tube, for instance a guide wire, and/or a biopsy needle.

As a result, a physician using the apparatus 10 for imaging an object of interest 22 arranged at the object receiving space 14 may locate the three-dimensional position p of the catheter and/or the guide wire within the object receiving space 14 and/or within the object of interest 22. Consequently, the physician may guide the catheter and/or guide wire along a three-dimensional roadmap of veins, in case the object of interest 22 is a human.

In an example, the X-ray source 16 may provide incoherent X-ray radiation 18. In order to provide coherent X-ray radiation to the object receiving space 14, a first grating 30 may be provided.

In an example, the first grating 30 is formed by an absorption grating or a phase grating. In a further example, the first grating may be formed by a combined absorption and phase grating. An example of the first grating 30 is shown in Fig. 3.

In an example, a grating, in particular the first grating 30, may also be referred to as an X-ray grating and/or an X-ray absorption grating.

In an example, the first grating 30 may also be referred as to the source grating or G0.

As shown in Fig. 3, the first grating 30 may be arranged between the X-ray source 16 and the object receiving space 14.

In an example, the first grating 30 may be configured to produce at least partly coherent X-ray radiation.

In an example, the first grating 30 may form the interferometer 24.

Alternatively, a second grating 32 shown in Fig. 1, arranged between the object receiving space 14 and detector 20, may form the interferometer 24.

In an example, the second grating 32 is formed by a phase grating or an absorption grating. In a further example, the second grating may be formed by a combined phase and absorption grating.

In an example, the second grating 32 may also refer to as G1.

In an example, the second grating 32 may be configured to generate a phase-shift between adjacent X-ray beams.

In a further example, a third grating 34 is arranged between the second grating 32 and the detector 20.

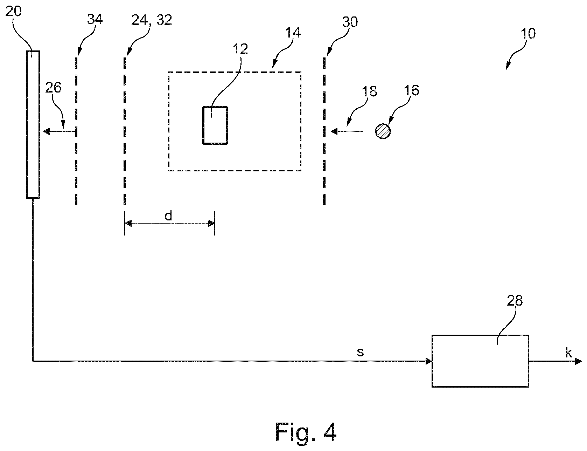

In an example, the third grating 34 is formed by an absorption grating or a phase grating. In a further example, the third grating may be formed by a combined absorption and phase grating. An example of the third phase grating 34 is shown in Fig. 4.

The third grating 34 may be configured as an analyzer grating.

In an example, the third grating 34 may be also referred to as the analyser grating or G2.

In an example, the third grating 34 may be configured to analyze the phase shift information resulting from an object of interest 22, which may be arranged at the object receiving space 14.

In an example, the X-ray radiation 18 provided by the X-ray source may impinge on the first grating 30, in order to provide at least partly coherent X-ray radiation to the object receiving space 14. In case an object 12 is arranged at the object receiving space 14, the at least partly coherent X-ray radiation provided to the object receiving space 14 may impinge on the object of interest 22. Further, the at least partly coherent X-ray radiation may also impinge on the interventional device 12, which may be arranged at the object receiving space 14, too. As a result, the first grating 30 and/or the object of interest 22 and/or the interventional device 12 may influence the X-ray radiation 18 provided by the X-ray source 16. After leaving the object receiving space 14, the X-ray radiation may impinge on the second grating 32 and/or the third grating 34, before the resulting X-ray radiation 26 impinges on the detector 20. Accordingly, the gratings 30, 32, 34, the object of interest 22 and/or the interventional device 12 may influence the X-ray radiation 18 provided by the X-ray source 16, before it impinges at the X-ray detector 20.

The X-ray detector 20 is configured to detect the X-ray radiation 26 influenced by the interferometer 24. On the basis of the detected X-ray radiation 26, the X-ray detector 20 may further be configured to provide a detector signal s. The detector signal s may indicate an image of the object receiving space 14 and/or any element arranged in the object receiving space 14, in particular an object of interest 22 and/or the interventional device 12.

The X-ray detector 20 and the processing unit 28 may be connected via a signal line, in order to provide the detector signal s to the processing unit 28.

The processing unit 28 is configured to identify, based on the detector signal s, the interventional device 12 within the object receiving space 14.

Object identification of an object at an image, in particular of the interventional device 12, is known in the state of the art. In particular, catheter localisation is known from C. Haase et al., "3D ablation catheter localisation using individual C-arm X-ray projection", Phys. Med. Biol. 59, 6959-6977, 2014. Accordingly, the interventional device 12 may be identified on the basis of the detector signal s. The detection may be performed continuously.

In an example, the identification of the interventional device 12 may relate to an identification of a two-dimensional position within an image plane corresponding to a view direction of the X-ray radiation provided by the X-ray source 16 to the X-ray detector 20.

In an example, the identification of the interventional device 12 may relate to an identification of a dimension of the interventional device 12, a shape of the interventional device 12 and/or any other two-dimensional geometrical information of the interventional device 12.

In an example, the processing unit 28 may be configured to calculate, on the basis of the detector signal s, an X-ray absorption image signal, a phase contrast image signal and/or a dark-field image signal.

In an example, the X-ray absorption image signal may represent an absorption image of the object receiving space 14, in particular of an object of interest 22 and/or an interventional device 12 arranged at the object receiving space 14.

In an example, the phase-contrast image signal may relate to a differential phase-contrast image of the object receiving space 14, in particular of an object of interest 22 and/or an interventional device 12 arranged at the object receiving space 14.

In an example, the dark-field image signal may relate to a dark-field image of the object receiving space 14, in particular of an object of interest 22 and/or an interventional device 12 arranged at the object receiving space 14.

In an example, the dark-field image signal may also refer to as the visibility loss signal.

In an example, the visibility loss signal caused by the interventional device 12 preferably relates to the respective component of the visibility loss signal indicating the interventional device 12 at the object receiving space 14.

It is to be noted, that the visibility loss signal in the further context preferably relates to the visibility loss signal of the interventional device 12.

In an example, the visibility loss signal may relate to a normalized difference between a maximum signal amplitude of the detector signal s and a minimum signal amplitude of the detector signal s, or vice versa.

In an example, the distance d between the interventional device 12, in case it is arranged at the object receiving space 14, and the first grating 30 as the interferometer 24 is a function of the visibility loss signal.

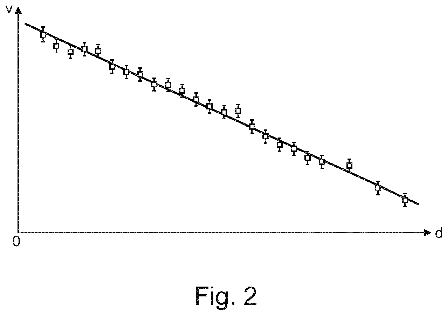

In an example, Fig. 2 schematically shows a relation between the distance d, namely a distance d between the first grating 32 and the interventional device 12 of the object receiving space 14 as a function of the visibility loss signal v.

In an example, the value of the visibility loss signal v approximately increases with a decrease of the distance d, and vice versa.

In an example, the distance d may be assumed as inverse proportional to the visibility loss signal v.

In an example, the visibility loss signal depends on the distance signal d. Preferably, the relation between the visibility loss signal and the distance signal d may be bijective.

In an example, the position signal k calculated by the process signal 28 may relate to a one-dimensional depth information of the interventional device 12 in a direction from the X-ray source 16 to the detector 20.

In an example, the position p, in particular formed by the distance d, may relate to an absolute and/or relative geometrical information of the interventional device 12.

In an example, the position signal k representing a position p of the interventional device 12 within the object receiving space 14 may be used to calculate a three-dimensional position of the interventional device 12 within the object receiving space 14.

In Fig. 4, another exemplary embodiment of the apparatus 10 is schematically shown.

In an example, the apparatus 10 comprises the three gratings 30, 32, 34 as set forth above. Thus, the apparatus 10 may comprise the first grating 30, the second grating 32 and the third grating 34.

In an example, the second grating 32 may form the interferometer 24 of the apparatus 10.

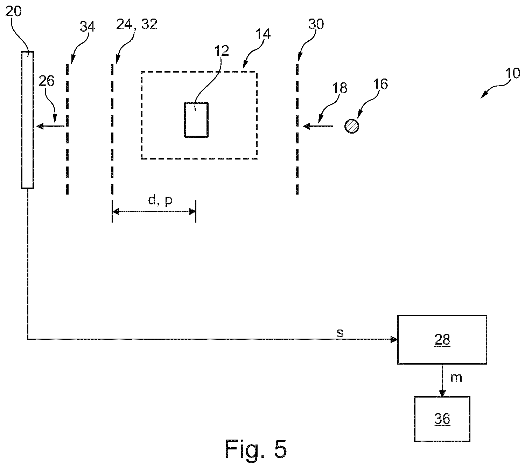

In Fig. 5, another exemplary embodiment of the apparatus 10 is schematically shown.

According to an example, the apparatus 10 may further comprise a display unit 36. Further, the processing unit 28 may be configured to calculate, based on the detector signal s, an image signal representing at least one part of the object of interest 22, which may be arranged at the object receiving space 14. Further, the processing unit 28 may be configured to calculate, based on the image signal and the position signal k, a display signal m representing the image of the image of the object of interest 22 and a notation of the position p of the interventional device 12. Furthermore, the display unit 36 may be configured to display a display image based on the display signal m.

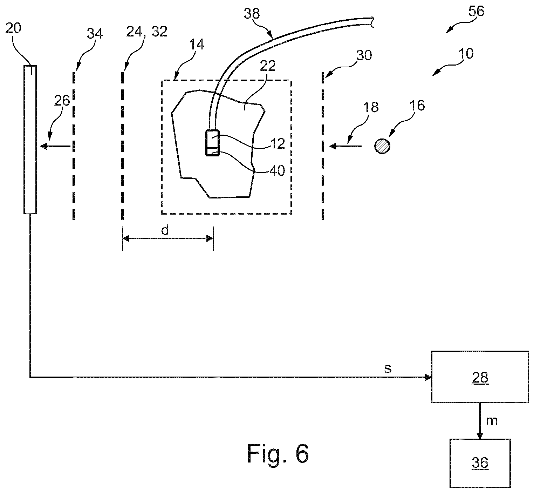

Fig. 6 schematically shows the apparatus 10, wherein an object of interest 22 is arranged at the object receiving space 14. The interventional device 12 may be arranged at the object of interest 22. The interventional device 12 may refer to an end section of a catheter 38. In a further example, the interventional device 12 is formed by the catheter 38.

As a result, the position signal k represents position information, in particular depth information, of the interventional device 12. This information may be recognizable by a notation at the display image, in particular by a colour coding, a numerical depiction, a slider bar and/or any other suitable form of an optical representation.

As a further result, a physician may recognize from the display image a two-dimensional, tomographical representation of at least a part of the object of interest 22 and/or the interventional device 12. Further, the physician may receive from the display image information about a third dimension of the position of the interventional device 12. Correspondingly, the physician may recognize a three-dimensional position of the interventional device 12 from the display image.

According to an example, the processing unit 28 may be configured to calculate, based on visibility loss signal, a distance signal representing a distance p between a section 40 of the interventional device 12 and the interferometer 24. Further, the processing unit 28 may be configured to calculate, based on the visibility loss signal, a distance signal representing a distance between a section 40 of the interventional device 12 and an element fixed to the interferometer 24. Furthermore, the processing unit 28 may be configured to calculate, based on the distance signal, the position signal k representing the position p of the interventional device 12 within the object receiving space 14.

As a result, a physician may receive further depth information about a particular section 40 of the interventional device 12 during an interventional procedure. The section 40 of the interventional device 12 may be of special interest and therefore may provide a higher interest of being tracked.

In an example, a heater, an ultrasonic element and/or any other actuator may be arranged at the section 40 of the interventional device 12.

As a result, for instance the heater can be tracked when being guided through the object of interest 22 at the object receiving space 14.

Calculating the distance signal may be interpreted as a sub-feature of calculating the position signal k. Accordingly, the configuration of the processing unit 28 for calculating the position signal may be interpreted as a sub-configuration of the configuration of the processing unit 28 to calculate the position signal.

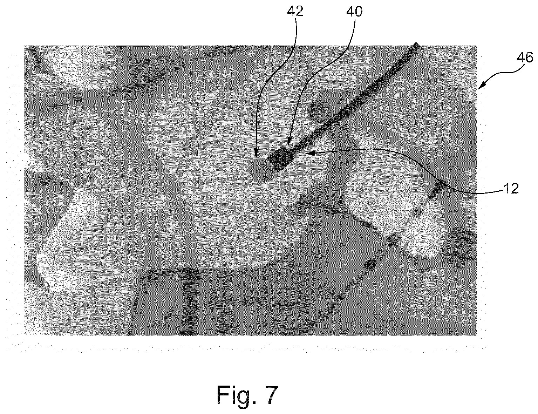

Fig. 7 exemplarily shows at least a part of a display image 46. The display image 46 may represent an absorption image of at least a part of an object of interest 22. Further, the display image 46 exemplarily shows a representation of the interventional device 12.

According to an example of the present invention, the notation of the position comprises a highlight-notation 42. Further, the processing unit 28 may be configured to determine a colour of the highlight-notation 42 based on the position signal k. Furthermore, the processing unit 28 may be configured to calculate the display signal m, such that the highlight-notation 42 highlights at least a part of the imaged interventional device 12 at the display image 46 with the colour determined for the highlight-notation 42.

As a result, the interventional device 12 imaged at the display image 46 may be highlighted, such that a physician can immediately identify the position p of the interventional device 12. Corresponding depth information of the interventional device 12 may be indicated by the colour of the highlight-notation 42. Accordingly, a physician can directly receive from the display image 46 a three-dimensional position-information of the interventional device 12, in particular with respect to the object of interest 22 and/or the object receiving space 14.

In an example, the notation is at least partly transparent.

As an effect, the display image 46 may show the at least a part of the object of interest 22, while highlighting the notation.

In an example, the notation has a form of a dot, a circle or any other geometrical form for optical recognition.

In an example, the dimension of the notation of the position corresponds to the value of the position, in particular the depth, of the interventional device 12.

As a result, the shown dimension of the interventional device 12 may indicate a depth of the interventional device 12. If the interventional device 12 is closer to the detector 20, the geometrical dimensions of the interventional device 12 may be represented larger at the display image than in case the interventional device 12 is arranged further away from the detector 20.

As a result, a physician can directly interpret from the display image 46 an approximate depth of the interventional device 12 within the object of interest 22.

In an example, an exchange function between the position signal k and a colour of the notation, in particular the highlight-notation 42, may be predefined. In particular, the image function may be set by predefined colours for predefined, distinct ranges of possible values of the position signal k.

In an example, the highlight-notation 42 highlights (just) a section 40, in particular a tip section, of the interventional device 12.

The section 40 of the interventional device 12 may comprise a sub-device of higher relevance. In particular, the section 40 of the interventional device 12 may comprise a heater. Accordingly, highlighting the section 40 of the interventional device 12 may indicate relevant information at the display image 46.

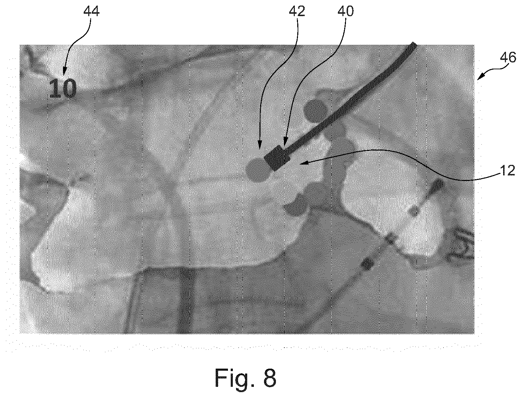

Fig. 8 exemplarily shows a further example of a display image 46.

According to an example of the present invention, the notation of the position comprises a number-notation 44 representing a value of the position p of the interventional device 12. The processing unit 28 may be configured to calculate the display signal m, such that the display image 46 comprises the number-notation 44.

As a result, the value of the position p of the interventional device 12, in particular its depth, may be directly shown at the display image 46.

As a further result, a physician may directly recognize at the display image 46 a depth information about the interventional device 12. Consequently, a three-dimensional position information of the interventional device 12, and therefore a direct localisation of the interventional device 12, in particular an object of interest 22, may be recognized.

In an example, the value of the position p of the interventional device 12 may be displayed at a corner and/or side section of the display image 46.

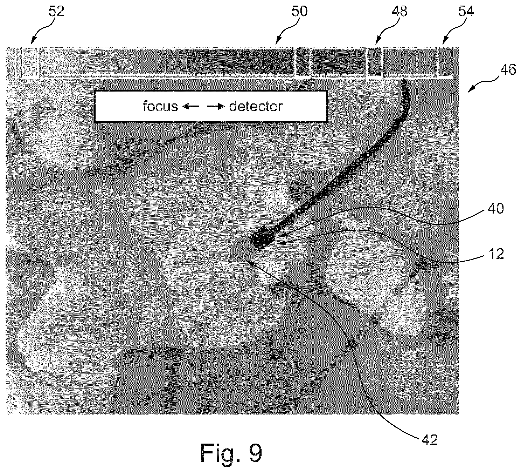

Fig. 9 shows another example of the display image 46.

According to a further example of the present invention, the notation of the position comprises a slider 48 and a slider bar 50. The slider bar 50 may represent a scale between a predefined minimum value 52 of the position signal k and a predefined maximum value 54 of the position signal k. The processing unit 28 may be configured to determine, on the basis of the position signal k, a slider position of the slider 48 at the slider bar 50. The processing unit 28 may be configured to calculate the display signal m, such that the display image also shows the slider bar 50 and the slider 48 at the slider position.

As an effect, the position of the slider 48 at the slider bar 50 may directly indicate a position, in particular a depth, of the interventional device 12 at the object receiving space 14 and/or the object of interest 22. Consequently, a physician may receive with a look at the display image quantitative information about the depth d of the interventional device 12, in particular of the section 40 of the interventional device 12.

According to an example of the present invention, the processing unit 28 may be configured to receive a spot signal. The processing unit 28 may be further configured to save, for each time of a reception of the spot signal, a position p of the section 40 of the interventional device 12 and the colour of the highlight-notation 42 for the section 40 as an interventional device spot. Further, the processing unit 40 may be configured to calculate the display image, such that the display image comprises each interventional device spot.

As an effect, the display image 46 may comprise several highlight-notations 42. Each highlight-notation 42 may be arranged at the position p of the interventional device 12 at the time, where the spot signal was received. Thus, a physician may mark the relevant positions p of the highlight-notations 42 to keep track of passed interventional device positions p, in particular of its section 40.

In an example, the section 40 of the interventional device 12 may relate to a tip section of the interventional device 12. In a further example, the apparatus 10 may comprise an input interface to receive the spot signal. For instance, a physician using the apparatus 10 may use the interface to send the spot signal to the apparatus 10.

In an example, the spot signal may be automatically generated, for instance when a heater, an ultrasonic element or another actuator is initiated at the interventional device 12.

According to an example of the present invention, an X-ray system 56 for determining a position p of an interventional device 12 within an object receiving space 14 is provided. Fig. 6 schematically shows an exemplary embodiment of the system 56.

According to the example, the system 56 comprises an apparatus 10 according to any of the preceding examples, and an interventional device 12. The interventional device 12 comprises a structure having a structure size z of less than twice of a grating pitch of a grating of the interferometer 24.

As a result, the interventional device 12 may cause a high contrast at the dark-field image signal component of the detector signal s and/or at the visibility loss signal. Thus, the interventional device 12 may be well identified on the basis of the visibility loss signal.

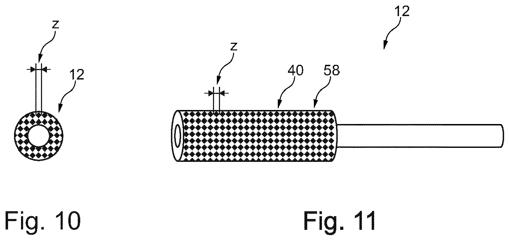

Fig. 10 schematically shows a cross-section of the interventional device 12. A structure of the interventional device 12 is indicated.

In an example, the interventional device 12 comprises at least at its surface a microstructure as the structure with the structure size z.

Fig. 11 schematically shows a perspective view at an section 40 of the interventional device 12. The structure having the structure size z is also indicated.

According to an example of the present invention, the interventional device 12 may comprise the structure having the structure size z at the section 40 of the interventional device 12.

In an example, the section 40 of the interventional device 12 may relate to a tip section of the interventional device 12. As a result, the section 40, in particular the tip section, of the interventional device 12 may be well identified.

According to an example of the present invention, the interventional device 12 comprises a sheath 58 for forming the structure having the structure size z. The sheath 58 may comprise an X-ray absorption characteristic, that has a maximum variance of 20% to an X-ray absorption characteristic of water.

As a result, the interventional device 12, in particular its sheath 58 at the section 40, has a very low influence on the absorption image signal component of the detector signal s. Thus, the absorption image signal component may be used to map the object of interest 22 with very low irritation of the interventional device 12.

In an example, the sheath 58 may form the structure having the structure size z. Accordingly, the sheath 58 of the interventional device 12 may form a structure, which can be well identified on the basis of the visibility loss signal.

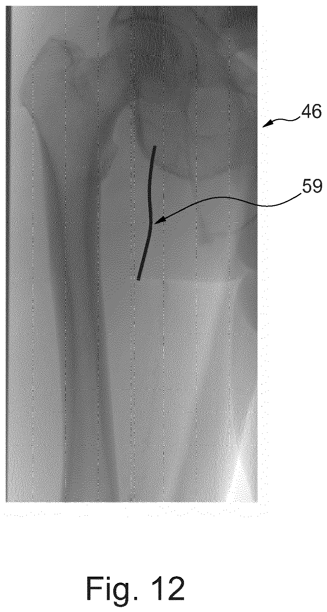

Fig. 12 exemplarily indicates a further example of a display image 46, wherein an exemplary representation 59 of the interventional device 12 is schematically indicated.

According to an example of the present invention, the processing unit 28 may be configured to calculate, based on the absorption-component of the detector signal s and/or a phase-contrast-component of the detector signal s, an image signal representing at least a part of the object of interest 22 at the object receiving space 14. The processing unit 28 may further be configured to calculate, based on identified interventional device 12, a device signal representing the interventional device 12. The processing unit 28 may further be configured to calculate the display signal m on the base of the image signal and the device signal, such that the interventional device 12 is represented as an overlay to the representation of the at least on part of the object of interest 22 at the display image 46.

As an effect, the absorption characteristic of the interventional device 12 may have a low effect at the visibility loss signal. Thus, the interventional device 12 can be identified and/or registered based on the visibility loss signal and indicated by the display signal as an overlay to the absorption image.

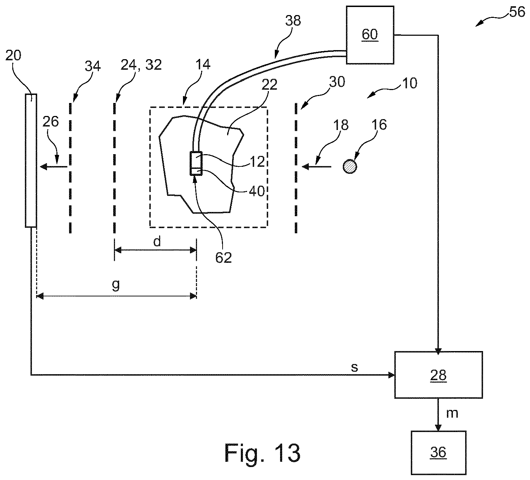

According to an example of the present invention, which is exemplarily shown in Fig. 13, an X-ray system 56 is provided, further comprising a control unit 60 for controlling the interventional device 12. The interventional device 12 may comprise an actuator 62 at a section 40 of the interventional device 12. The control unit 60 may be configured to activate the actuator 62. The control unit 60 may be configured to send a spot signal to the processing unit 28 upon the activation of the actuator 62.

In an example, the actuator 62 may be formed by a heater, an ultrasonic transducer and/or any other human tissue impact actuator.

In an example, the section 40 of the interventional device 12 may be its tip section.

As a result, a spot signal received by the processing unit 28 may create an interventional device spot, in particular comprising a highlight-notation at the current position of the interventional device 12. The interventional device spots may remain at the display image.

As a further result, a physician may keep track of the past three-dimensional positions p of the interventional device 12, and in particular to keep track of the positions p of heat applications at the object of interest 22.

According to an example of the present invention, a method 64 for determining a position p of the interventional device 12 within an object receiving space 14 may be provided.

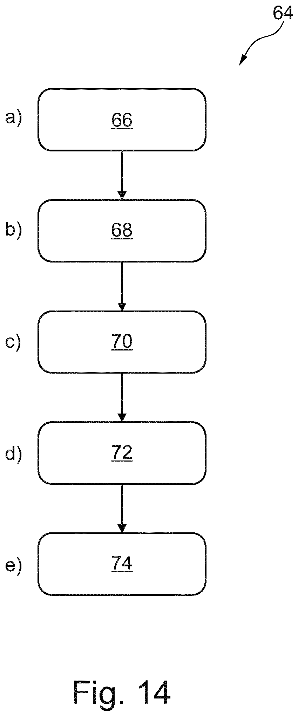

As shown in Fig. 14, the method 64 may comprise the following:
In a first emitting-step 66, also referred as to step a), an X-ray radiation 18 is emitted from the X-ray source 16 in direction to an X-ray detector 20, wherein an object receiving space 14 is arranged between the X-ray source 16 and the X-ray detector 20, and wherein an interferometer 24 is arranged between the object receiving space 14 and the X-ray detector 20 or the X-ray source 16.

In a second providing-step 68, also referred to as step b), a detector signal s, based on influenced X-ray radiation 26 detected by the detector 20, is provided.

According to a third identifying step 70, also referred to as step c), an interventional device 12 is identified within the object receiving space 14 based on the detector signal s.

In a fourth calculating-step 72, also referred to as step d), a visibility loss signal for the identified interventional device 12 is calculated based on the detector signal s.

According to a fifth calculating-step 74, also referred to as step e), a position signal k representing a position p of the interventional device 12 within the object receiving space 14 is calculated based on the visibility loss signal.

It is to be understood that, without repeating here all examples and explanations provided with reference to the apparatus and/or the system of the invention, the method of the invention is intended as being configured to provide corresponding method steps. Thus, all the above explanations and examples, although provided with reference to the apparatus and/or the system, are also intended to be implemented analogously by the method.

According to a further example of the present invention, a computer program element is provided, which, when being executed by a processing unit 28, is adapted to carry out the method 64 described above.

According to a further example of the present invention, a computer-readable medium having stored thereon a program element is provided, which, when being executed by a processing unit 28, is adapted to carry out the method 64 described above.

## Claims

1. An X-ray imaging apparatus (10) for determining a position (p) of an interventional device (12) within an object receiving space (14), comprising:
- an X-ray source (16) for generating an X-ray radiation (18);
- an X-ray detector (20);
- the object receiving space for arranging an object of interest (22) for X-ray imaging, wherein the object receiving space is arranged between the X-ray source and the X-ray detector; and
- an interferometer (24) for creating interference pattern, wherein the interferometer is arranged between the object receiving space and the X-ray source or the object receiving space and the X-ray detector;
wherein the X-ray detector is configured to detect X-ray radiation (26) influenced by the interferometer and in response thereto provide a detector signal (s); and
wherein the imaging apparatus further comprises:
- a processing unit (28) configured
(i) to identify, based on the detector signal, the interventional device within the object receiving space;
(ii) to calculate, based on the detector signal, a visibility loss signal for the identified interventional device; and
(iii) to calculate, based on the visibility loss signal, a position signal representing the position of the interventional device within the object receiving space.

2. Apparatus according to claim 1, further comprising a display unit (36), wherein the processing unit is configured to calculate, based on the detector signal, an image signal representing at least one part of the object of interest, and to calculate, based on the image signal and the position signal, a display signal (m) representing the image of the object of interest and a notation of the position of the interventional device; and wherein the display unit is configured to display a display image based on the display signal.

3. Apparatus according to any of the claims 1 to 2, wherein the processing unit is configured to calculate, based on the visibility loss signal, a distance signal representing a distance (d) between a section (40) of the interventional device and the interferometer or an element fixed thereto and to calculate, based on the distance signal, the position signal representing the position of the interventional device within the object receiving space.

4. Apparatus according to any of the claims 2 to 3, wherein the notation of the position comprises a highlight-notation (42); wherein the processing unit is configured to determine a colour of the highlight-notation based on the position signal; and wherein the processing unit is configured to calculate the display signal, such that the highlight-notation highlights at least a part of the imaged interventional device at the display image with the colour determined for the highlight-notation.

5. Apparatus according to any of the claims 2 to 4, wherein the notation of the position comprises a number-notation (44) representing a value of the position of the interventional device; and wherein the processing unit is configured to calculate the display signal, such that the display image comprises the number-notation.

6. Apparatus according to any of the claims 2 to 5, wherein the notation of the position comprises a slider (48) and a slider bar (50); wherein the slider bar represents a scale between a predefined minimum value of the position signal and a predefined maximum value of the position signal; wherein the processing unit is configured to determine, on the basis of the position signal, a slider position of the slider at the slider bar; and wherein the processing unit is configured to calculate the display signal, such that the display image also shows the slider bar and the slider at the slider position.

7. Apparatus according to any of the claims 2 to 6, wherein the processing unit is configured to receive a spot signal; wherein the processing unit is configured to save, for each time of a reception of the spot signal, a position of the section of the interventional device and the colour of the highlight-notation for the section as an interventional device spot; and wherein the processing unit is configured to calculate the display image, such that the display image comprises each interventional device spot.

8. An X-ray system (56) for determining a position of an interventional device within an object receiving space, comprising: an apparatus according to any of the claims 1 to 7; and the interventional device; wherein the interventional device comprises a structure comprising a structure size (z) of less than twice of a grating pitch of a grating of the interferometer.

9. An X-ray system according to any claim 8, wherein the interventional device comprises the structure having the structure size at a section of the interventional device.

10. An X-ray system according to any of the claims 8 to 9, wherein the interventional device comprises a sheath (58) for forming the structure having the structure size; and wherein the sheath comprises an X-ray absorption characteristic, that has a maximum variance of 20% to an X-ray absorption characteristic of water.

11. An X-ray system according to any of the claims 8 to 10, wherein the processing unit is configured to calculate, based on an absorption-component of the detector signal and/or a phase-contrast-component of the detector signal, an image signal representing at least one part of the object of interest at the object receiving space; wherein the processing unit is configured to calculate, based on the identified interventional device, a device signal representing at least one part of the interventional device; and wherein the processing unit is configured to calculate the display signal on the basis of the image signal and the device signal, such that the interventional device is represented as an overlay to the representation of the object of interest at the display image.

12. An X-ray system according to any of the claims 8 to 11, comprising a control unit (60) for controlling the interventional device; wherein the interventional device comprises an actuator (62) at a section of the interventional device; wherein the control unit is configured to activate the actuator; and wherein the control unit is configured to send a spot signal to the processing unit upon the activation of the actuator.

13. A method (64) for determining a position of an interventional device within an object receiving space, comprising the following steps:
a) emitting (66) an X-ray radiation from an X-ray source in direction to an X-ray detector, wherein an object receiving space is arranged between the X-ray source and the X-ray detector, and wherein an interferometer is arranged between the object receiving space and the X-ray detector or the object receiving space and the X-ray source;
b) providing (68) a detector signal based on X-ray radiation detected by the detector, wherein the detected X-ray radiation is influenced by the interferometer and/or an object of interest arrangeable at the object receiving space;
c) identifying (70) with a processing unit , based on the detector signal, an interventional device within the object receiving space;
d) calculating (72) with the processing unit, based on the detector signal, a visibility loss signal for the identified interventional device; and
e) calculating (74) with the processing unit, based on the visibility loss signal, a position signal representing a position of the interventional device within the object receiving space.

14. A computer program element for controlling a device according to one of the claims 1 to 12, which, when being executed by the processing unit, is adapted to perform the method steps of claim 13.

15. A computer readable medium having stored the program element of claim 14.

## Patentansprüche

1. Ein Röntgen-Bildgebungsgerät (10) zum Ermitteln der Position (p) eines Interventionsgeräts (12) im Objektaufnahmebereich (14), das Folgendes umfasst:
- eine Röntgenquelle (16) zum Erzeugen von Röntgenstrahlung (18);
- einen Röntgendetektor (20);
- den Objektaufnahmebereich zum Anordnen des zu untersuchenden Objekts (22) für die Röntgenbildgebung, wobei sich der Objektaufnahmebereich zwischen der Röntgenquelle und dem Röntgendetektor befindet; und
- ein Interferometer (24) zum Erzeugen von Interferenzmustern, wobei sich das Interferometer zwischen dem Objektaufnahmebereich und der Röntgenquelle oder dem Objektaufnahmebereich und dem Röntgendetektor befindet;
wobei der Röntgendetektor die durch das Interferometer beeinflusste Röntgenstrahlung (26) erfasst und als Reaktion ein Detektorsignal (s) bereitstellt; und wobei das Bildgebungsgerät zudem Folgendes umfasst:
- eine Verarbeitungseinheit (28), die
(i) anhand des Detektorsignals das Interventionsgerät im Objektaufnahmebereich ermittelt;
(ii) anhand des Detektorsignals ein Visibilitätsverlustsignal für das ermittelte Interventionsgerät berechnet; und
(iii) anhand des Visibiltätsverlustsignals ein Positionssignal berechnet, das die Position des Interventionsgerät im Objektaufnahmebereich angibt.

2. Gerät gemäß Anspruch 1,
das zudem eine Anzeigeeinheit (36) umfasst,
wobei die Verarbeitungseinheit anhand des Detektorsignals ein Bildsignal berechnet, mit dem mindestens ein Teil des zu untersuchenden Objekts dargestellt wird. Zudem berechnet Sie anhand des Bild- und Positionssignals ein Anzeigesignal (m), um das Bild des zu untersuchenden Objekts sowie die Position des Interventionsgeräts darzustellen. Hierbei wird auf der Anzeigeeinheit anhand des Anzeigesignals ein Bild angezeigt.

3. Das Gerät gemäß einem der Ansprüche 1 und 2,
wobei die Verarbeitungseinheit anhand des Visibiltätsverlustsignals ein Abstandssignal berechnet, das den Abstand (d) zwischen einem Abschnitt (40) des Interventionsgeräts und dem Interferometer oder einem an diesem befestigten Element angibt. Zudem berechnet sie anhand des Abstandssignals das Positionssignal, das die Position des Interventionsgeräts im Objektaufnahmebereich angibt.

4. Das Gerät gemäß einem der Ansprüche 2 und 3,
wobei die Angabe der Position eine Hervorhebung (42) umfasst; und wobei die Verarbeitungseinheit beruhend auf dem Positionssignal die Farbe der Hervorhebung bestimmt; und wobei die Verarbeitungseinheit das Anzeigesignal so berechnet, dass die Hervorhebung zumindest einen Teil des abgebildeten Interventionsgeräts auf dem Bild in der für die Hervorhebung festgelegten Farbe darstellt.

5. Das Gerät gemäß einem der Ansprüche 2 bis 4,
wobei die Angabe der Position eine Zahl (44) umfasst, die dem Wert der Position des Interventionsgeräts entspricht;
und wobei die Verarbeitungseinheit das Anzeigesignal so berechnet, dass das Bild die Zahl umfasst.

6. Das Gerät gemäß einem der Ansprüche 2 bis 5,
wobei die Angabe der Position einen Schieberegler (48) und eine Schieberegler-Leiste (50) umfasst; und wobei die Schieberegler-Leiste einer Skala zwischen einem vordefinierten Minimalwert für das Positionssignal und einem vordefinierten Maximalwert für das Positionssignal entspricht; und wobei die Verarbeitungseinheit anhand des Positionssignals eine Schiebereglerposition des Schiebereglers in der Schieberegler-Leiste ermittelt; und wobei die Verarbeitungseinheit das Anzeigesignal so berechnet, dass im Bild zudem die Schieberegler-Leiste und der Schieberegler an der Schiebereglerposition angezeigt werden.

7. Das Gerät gemäß einem der Ansprüche 2 bis 6,
wobei die Verarbeitungseinheit ein Punktsignal empfängt; und wobei die Verarbeitungseinheit für jeden Empfangszeitpunkt des Punktsignals eine Position des Abschnitts des Interventionsgeräts sowie die Farbe der Hervorhebung für den Abschnitt als einen Interventionsgerätepunkt speichert;
und wobei die Verarbeitungseinheit das Bild so berechnet, dass dieses alle Punkte des Interventionsgeräts umfasst.

8. Ein Röntgensystem (56) zum Ermitteln der Position eines Interventionsgeräts im Objektaufnahmebereich, das Folgendes umfasst: ein Gerät gemäß einem der Ansprüche 1 bis 7; und das Interventionsgerät; wobei das Interventionsgerät eine Struktur mit der Strukturgröße (z) aufweist, die weniger als das Zweifache der Rasterteilung des Interferometer-Rasters beträgt.

9. Ein Röntgensystem gemäß Anspruch 8,
wobei das Interventionsgerät eine Struktur umfasst, die an einem Abschnitt des Interventionsgeräts eine Strukturgröße aufweist.

10. Ein Röntgensystem gemäß einem der Ansprüche 8 und 9,
wobei das Interventionsgerät eine Hülle (58) zum Bilden der Struktur mit der Strukturgröße umfasst;
und wobei die Hülle eine Röntgenabsorptionscharakteristik aufweist, die einer maximalen Abweichung von 20 % zur Röntgenabsorptionscharakteristik von Wasser entspricht.

11. Ein Röntgensystem gemäß einem der Ansprüche 8 und 10,
wobei die Verarbeitungseinheit anhand einer Absorptionskomponente des Detektorsignals und/oder einer Phasenkontrast-Komponente des Detektorsignals ein Bildsignal berechnet, das mindestens einem Teil des zu untersuchenden Objekts im Objektaufnahmebereich entspricht;
und wobei die Verarbeitungseinheit anhand des ermittelten Interventionsgeräts ein Gerätesignal berechnet, das mindestens einem Teil des Interventionsgeräts entspricht; und wobei die Verarbeitungseinheit das Anzeigesignal anhand des Bildsignals und des Gerätesignals so berechnet, dass das Interventionsgerät im Bild als Überlagerung des darstellten zu untersuchenden Objekts angezeigt wird.

12. Ein Röntgensystem gemäß einem der Ansprüche 8 und 11,
das eine Steuerungseinheit (60) zum Steuern des Interventionsgeräts umfasst; wobei das Interventionsgerät an einem seiner Abschnitt einen Aktuator (62) umfasst; und wobei die Steuerungseinheit den Aktuator aktiviert; und wobei die Steuereinheit beim Aktivieren des Aktuators ein Punktsignal an die Verarbeitungseinheit sendet.

13. Eine Methode (64) zum Ermitteln der Position eines Interventionsgeräts im Objektaufnahmebereich, die folgende Schritte umfasst:
a) Emittieren (66) einer Röntgenstrahlung von der Röntgenquelle in Richtung des Röntgendetektors, wobei sich zwischen der Röntgenquelle und dem Röntgendetektor ein Objektaufnahmebereich befindet, und wobei sich zwischen dem Objektaufnahmebereich und dem Röntgendetektor oder dem Objektaufnahmebereich und der Röntgenquelle ein Interferometer befindet;
b) Bereitstellen (68) eines Detektorsignals beruhend auf der vom Detektor erkannten Röntgenstrahlung, wobei die erkannte Röntgenstrahlung vom Interferometer und/oder vom zu untersuchenden Objekt im Objektaufnahmebereich beeinflusst wird;
c) Ermitteln (70) eines Interventionsgeräts im Objektaufnahmebereich mit einer Verarbeitungseinheit beruhend auf dem Detektorsignal;
d) Berechnen (72) eines Visibilitätsverlustsignals für das ermittelte Interventionsgerät mit der Verarbeitungseinheit beruhend auf dem Detektorsignal; und
e) Berechnen (74) eines Positionssignals, das die Position des Interventionsgeräts im Objektaufnahmebereich angibt, mit der Verarbeitungseinheit beruhend auf dem Visibilitätsverlustsignal.

14. Ein Computerprogrammelement zum Steuern eines Geräts gemäß einer der Ansprüche 1 bis 12, das beim Ausführen durch eine Verarbeitungseinheit die Schritte der Methode gemäß Anspruch 13 durchführt.

15. Ein von einem Computer lesbares Medium, auf dem das Programmelement gemäß Anspruch 14 gespeichert wird.

## Revendications

1. Appareil d'imagerie par rayons X (10) permettant de déterminer une position (p) d'un dispositif interventionnel (12) dans un espace de réception d'objet (14), comprenant :
- une source de rayons X (16) pour générer un rayonnement des rayons X (18) ;
- un détecteur de rayons X (20) ;
- un espace de réception d'objet pour disposer un objet d'intérêt (22) pour l'imagerie par rayons X, dans lequel l'espace de réception d'objet est agencé entre la source de rayons X et le détecteur de rayons X ; et
- un interféromètre (24) pour créer un motif d'interférence, dans lequel dans l'interféromètre est disposé entre l'espace de réception d'objet et la source de rayons X ou l'espace de réception d'objet et le détecteur de rayons X ;
dans lequel le détecteur de rayons X est conçu pour détecter un rayonnement des rayons X (26) influencé par l'interféromètre et en réponse à celui-ci, pour fournir un signal de détecteur (S) ; et
dans lequel l'appareil d'imagerie comprend en outre :
- une unité de traitement (28) conçue
(i) pour identifier, en fonction du signal de détecteur, le dispositif interventionnel dans l'espace de réception d'objet ;
(ii) pour calculer, en fonction du signal de détecteur, un signal de perte de visibilité pour le dispositif interventionnel identifié ; et
(iii) pour calculer, en fonction du signal de perte de visibilité, un signal de position représentant la position du dispositif interventionnel dans l'espace de réception d'objet.

2. Appareil selon la revendication 1, comprenant en outre une unité d'affichage (36), dans lequel l'unité de traitement est conçue pour calculer, en fonction du signal de détecteur, un signal d'image représentant au moins une partie de l'objet d'intérêt, et pour calculer, en fonction du signal d'image et du signal de position, un signal d'affichage (m) représentant l'image de l'objet d'intérêt et une notation de la position du dispositif interventionnel ; et dans lequel l'unité d'affichage est conçue pour afficher une image d'affichage en fonction du signal d'affichage.

3. Appareil selon l'une quelconque des revendications 1 à 2, dans lequel l'unité de traitement est conçue pour calculer, en fonction du signal de perte de visibilité, un signal de distance représentant une distance (d) entre une section (40) du dispositif interventionnel et l'interféromètre ou un élément, lequel est fixé à celui-ci et pour calculer, en fonction du signal de distance, le signal de position représentant la position du dispositif interventionnel dans l'espace de réception d'objet.

4. Appareil selon l'une quelconque des revendications 2 à 3, dans lequel la notation de la position comprend une notation de surbrillance (42) ;
dans lequel l'unité de traitement est conçue pour déterminer une couleur de la notation de surbrillance en fonction du signal de position ; et
dans lequel l'unité de traitement est conçue pour calculer le signal d'affichage de telle sorte que la notation de surbrillance met en évidence au moins une partie du dispositif interventionnel imagée au niveau de l'image d'affichage à l'aide de la couleur déterminée pour la notation de surbrillance.

5. Appareil selon l'une quelconque des revendications 2 à 4, dans lequel la notation de la position comprend une notation numérique (44) représentant une valeur de la position du dispositif interventionnel ; et
dans lequel l'unité de traitement est conçue pour calculer le signal d'affichage de telle sorte que l'image d'affichage comprend la notation numérique.

6. Appareil selon l'une quelconque des revendications 2 à 5, dans lequel la notation de la position comprend un curseur (48) et une barre de curseur (50) ,
dans lequel la barre de curseur représente une échelle entre une valeur minimale prédéfinie du signal de position et une valeur maximale prédéfinie du signal de position ;
dans lequel l'unité de traitement est conçue pour déterminer, en fonction du signal de position, une position de curseur du curseur au niveau de la barre de curseur, et dans lequel l'unité de traitement est conçue pour calculer le signal d'affichage de telle sorte que l'image d'affichage montre également la barre de curseur et le curseur à la position du curseur.

7. Appareil selon l'une quelconque des revendications 2 à 6, dans lequel l'unité de traitement est conçue pour recevoir un signal ponctuel ;
dans lequel l'unité de traitement est conçue pour sauvegarder, pour chaque temps d'une réception du signal ponctuel, une position de la section du dispositif interventionnel et la couleur de la notation de surbrillance pour la section en tant que point de dispositif interventionnel ; et
dans lequel l'unité de traitement est conçue pour calculer l'image d'affichage de telle sorte que l'image d'affichage comprend chaque point de dispositif interventionnel.

8. Système à rayons X (56) permettant de déterminer une position d'un dispositif interventionnel dans un espace de réception d'objet, comprenant :
un appareil selon l'une quelconque des revendications 1 à 7 ; et
le dispositif interventionnel ;
dans lequel le dispositif interventionnel comprend une structure présentant une taille de structure (Z) inférieure à deux fois d'un pas de réseau d'un réseau de l'interféromètre.

9. Système à rayons X selon l'une quelconque des revendications 8, dans lequel le dispositif interventionnel comprend la structure présentant :
une taille de structure à une section du dispositif interventionnel.

10. Système à rayons X selon l'une quelconque des revendications 8 à 9, dans lequel le dispositif interventionnel comprend une enveloppe (58) pour former la structure présentant la taille de structure ; et dans lequel l'enveloppe comprend une caractéristique d'absorption des rayons X, laquelle présente une variance maximale de 20 % par rapport à une caractéristique d'absorption des rayons X de l'eau.

11. Système à rayons X selon l'une quelconque des revendications 8 à 10, dans lequel l'unité de traitement est conçue pour calculer, en fonction d'une composante d'absorption du signal de détecteur et/ou d'une composante de contraste de phase du signal de détecteur, un signal d'image représentant au moins une partie de l'objet d'intérêt dans l'espace de réception d'objet ;
dans lequel l'unité de traitement est conçue pour calculer, en fonction du dispositif interventionnel identifié, un signal de dispositif représentant au moins une partie du dispositif interventionnel ; et dans lequel l'unité de traitement est conçue pour calculer le signal d'affichage en fonction du signal d'image et du signal de dispositif, de telle sorte que le dispositif interventionnel est représenté en tant qu'une superposition à la représentation de l'objet d'intérêt sur l'image d'affichage.

12. Système à rayons X selon l'une quelconque des revendications 8 à 11, comprenant une unité de commande (60) pour commander le dispositif interventionnel ;
dans lequel le dispositif interventionnel comprend un actionneur (62) dans une section du dispositif interventionnel ;
dans lequel l'unité de commande est conçue pour activer l'actionneur ; et
dans lequel l'unité de commande est conçue pour envoyer un signal ponctuel à l'unité de traitement lors de l'activation de l'actionneur.

13. Procédé (64) de détermination d'une position d'un dispositif interventionnel dans un espace de réception d'objet, comprenant les étapes suivantes :
a) l'émission (66) d'un rayonnement des rayons X depuis une source de rayons X en direction vers un détecteur de rayons X, dans lequel un espace de réception d'objet est agencé entre la source de rayons X et le détecteur de rayons X, et dans lequel un interféromètre est agencé entre l'espace de réception d'objet et le détecteur de rayons X ou l'espace de réception d'objet et la source de rayons X ;
b) la fourniture (68) d'un signal de détecteur en fonction du rayonnement X détecté par le détecteur, dans lequel le rayonnement des rayons X détecté est influencé par l'interféromètre et/ou un objet d'intérêt pouvant être disposé dans l'espace de réception d'objet ;
c) l'identification (70) à l'aide d'une unité de traitement, en fonction du signal de détecteur, d'un dispositif interventionnel dans l'espace de réception d'objet ;
d) le calcul (72) à l'aide de l'unité de traitement, en fonction du signal de détecteur, d'un signal de perte de visibilité pour le dispositif interventionnel identifié ; et
e) le calcul (74) à l'aide de l'unité de trait-ment, en fonction du signal de perte de visibilité, d'un signal de position représentant une position du dispositif interventionnel dans l'espace de réception d'objet.

14. Élément de programme informatique permettant de commander un dispositif selon l'une quelconque des revendications 1 à 12, lequel, lorsqu'il est exécuté par l'unité de traitement, est apte à la mise en œuvre des étapes de procédé selon la revendication 13.

15. Support lisible par ordinateur comportant l'élément de programme selon la revendication 14 mémorisé.
